# EUROPEAN PATENT SPECIFICATION

(11) **EP 1 222 157 B1**
(45) Date of publication and mention of the grant of the patent: **28.04.2004**
(21) Application number: 00967905.1
(22) Date of filing: 20.10.2000
(51) Int. Cl.: C07C 67/38, C07C 69/54, C07C 69/618, C07C 69/533

(54) **PROCESS FOR THE CARBONYLATION OF AN ACETYLENICALLY UNSATURATED COMPOUND**
VERFAHREN ZUM CARBONYLIEREN EINER ACETYLENISCH UNGESÄTTIGTEN VERBINDUNG
PROCEDE DE CARBONYLATION D'UN COMPOSE INSATURE EN ACETYLENE

(30) Priority: 22.10.1999 EP 99308353
(43) Date of publication of application: 17.07.2002
(73) Proprietor: SHELL INTERNATIONALE RESEARCH MAATSCHAPPIJ B.V., 2596 HR Den Haag (NL)
(72) Inventor: DRENT, Eit, NL-1031 CM Amsterdam (NL); PRINGLE, Paul, Gerard, Bristol, Avon BS8 1TS (GB); PUGH, Robert, Ian, NL-1031 CM Amsterdam (NL)
(86) International application number: PCT/EP2000/010427
(87) International publication number: WO 2001/028972

(56) References cited:
- WO-A-94/21585
- WO-A-98/42717

## Description

The invention is related to a process for the carbonylation of an acetylenically unsaturated compound by reaction with carbon monoxide and a co-reactant in the presence of a catalyst system based on (a) a source of platinum: (b) a source of anions and (c) a diphosphine. The invention is especially directed to a process in which linear carbonylation products are selectively prepared.

In view of the fact that for a number of outlets the availability of linear, rather than branched carbonylated products would be desirable, e.g. in the preparation of components of detergent compositions, efforts have been made to increase the selectivity with respect to linear carbonylation products. WO-A-9421585 describes a process, wherein use is made of a catalyst system based on platinum, a bisphosphine ligand and a source of anions, typically a strong acid. A disadvantage of this process is that the activity of this catalyst system is somewhat low, being in the order of 20 to 200 mole product per mole Pt per hour for acetylene as reactant.

A process having a higher catalyst activity is known from WO-A-9720803. A carbonylation of acetylene with carbon monoxide and t-butyl alcohol is described using a catalyst system consisting of 1,2-P,P'-bis(9-phosphabicyclononyl)ethane as the ligand, platinum(II)tacetylacetonate)2 as the platinum source, methane sulphonic acid as the anion source, SnCl₂ and 2,5,8-tri-oxanonane.

A disadvantage of this process is that SnCl₂ has to be present in order to achieve an acceptable activity and selectivity. The use of SnCl₂ is not preferred because when performing this process in a continuous manner SnCl₂ will be lost due to alcoholysis. This is not desirable because new SnCl₂ has to be added and the tin alkoxyde formed will have to be discharged resulting in obvious environmental problems. Furthermore when tin alkoxyde is removed from the process by means of a purge additional catalyst will be lost. It is therefore highly desirable to perform such a carbonylation process in the absence of tin chloride compounds like SnCl₂.

US-A-5719313 describes the carbonylation of propyne with CO and methanol using a catalyst system consisting of palladium (II) acetate, bisphenyl(2-pyridyl)phosphine and methanesulfonic acid. A disadvantage of this process is that the mono phosphine ligand is consumed in the process by 'quarternization' of the ligand with the acrylate product. A further disadvantage of this process is that mostly branched products are formed.

EP-A-0441446 describes the carbonylation of ethene and the carbonylation of propyne with CO and a hydroxy group containing compound using a catalyst system consisting of palladium (II) acetate, a sulphonic acid, a bisphenyl(2-pyridyl) mono-phosphine and a tertiary amine as promoter. A disadvantage of this process is that a promoting compound in the form of a tertiary amine is necessary. A further disadvantage of this process is that mostly branched products are formed.

WO-A-9842717 describes the carbonylation of ethene with carbon monoxide and methanol in the presence of a catalyst system consisting of platinum (II) (acetylacetonate)2, 1,3-P,P'-di(2-phespha-1,3,5,7-tetramethyl-6,9,10-trioxatricyclo[3.3.1.1{3.7}decyl)propane and methanesulphonic acid. The publication does not disclose that acetylenically unsaturated compounds can be carbonylated using such a catalyst system.

The object of the present invention is a process for the carbonylation of acetylene to linear products, which process can be performed in the absence of SnCl₂. Such a process is described below. Process for the carbonylation of an acetylenically unsaturated compound by reaction with carbon monoxide and a co-reactant containing at least one mobile hydrogen atom in the presence of a catalyst system based on (a) a source of platinum; (b) a source of anions and (c) a diphosphine of the following formula

R¹>P-R²-PR³R⁴ (1)

wherein R² represents a covalent bridging group, R¹ represents a bivalent radical that together with the phosphorus atom to which it is attached is an optionally substituted 2-phospha-tricyclo[3.3.1.1{3,7}]decyl group or a derivative thereof in which one or more of the carbon atoms are replaced by heteroatoms ("2-PA" group), and wherein R³ and R⁴ independently represent univalent radicals of up to 20 atoms or jointly form a bivalent radical of up to 20 atoms.

With the above process the carbonylation can be performed with a good turn over rate, a high selectivity to the desired carbonylation products and in the absence of SnCl₂. It has furthermore been found that the ligand is stable over a prolonged period of time making it very suitable for use in a continuously operated commercial application.

Tricyclo[3.3.1.1{3,7}]decane is the systematic name for a compound more generally known as adamantane. Therefore, the optionally substituted 2-phospha-tricyclo-[3.3.1.1{3,7}]decyl group will be referred to as "2-PA" group (as in 2-phosphadamantyl group) throughout the specification.

The 2-PA group has preferably additional heteroatoms other than the 2-phosphorus atom in its skeleton. Suitable heteroatoms are oxygen and sulphur atoms. Suitably, these heteroatoms are found in the 6, 9 and 10 positions. The most preferred bivalent radical is the 2-phospha-1,3,5,7-tetramethyl-6,9,10-trioxadamantyl group. Preferably, the 2-PA group is substituted on one or more of the 1, 3, 5 or 7 positions with a monovalent radical R⁵ of up to 20 atoms. Typical examples of R⁵ include methyl, trifluoromethyl, ethoxy, phenyl, and 4-dodecylphenyl. More preferably, the 2-PA group is substituted on each of the 1, 3, 5 and 7 positions, suitably with identical radicals R⁵.

Each of the monovalent radicals R³ and R⁴ may independently be selected from (substituted) hydrocarbyl groups such as, for instance, methyl, phenyl, pyridyl, or o,o-di(t-butoxy)phenyl, and (substituted) heterohydrocarbyl groups such as, for instance, trimethylsilyl or alkoxy groups. Alternatively, R³ and R⁴ may together form a bivalent radical, such as 1,6-hexylene, 1,3 or 1,4-cyclooctylene. Preferably, R³ and R⁴ together with the phosphorus atom form a 2-PA group. Most preferably R² connects two identical 2-PA groups.

. The bridging group R² can be an organic bivalent group having up to 20 atoms. Examples of such bridging groups are ferrocenyl and nickelocenyle. Preferably R² has 2 to 4 atoms in the shortest chain of atoms directly connecting the two phosphorus atoms. Most preferably the number of atoms in the shortest chain of atoms directly connecting the two phosphorus atoms is 3. Preferably the atoms in the chain are carbon atoms. Examples of preferred bridging groups are ortho xylyl, ethylene and tri-methylene groups.

A suitable example of such a ligand is one according to the following general formula: 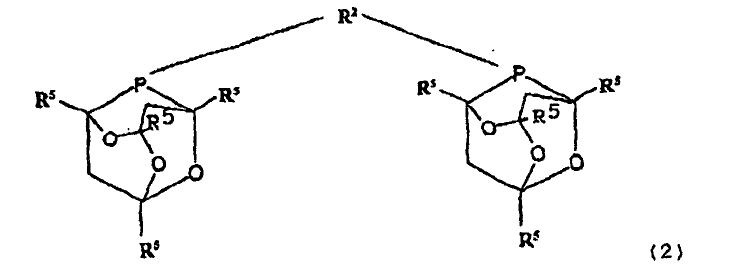 in which R⁵ and R² can be as describes above.

Examples of ligands which may be used in the process according to the invention are exemplified in the above cited WO-A-9842717. Most preferably 1,2-P,P'-di(2-phospha-1,3,5,7-tetramethyl-6,9,10-trioxatricyclo-[3.3.1.1{3.7}decyl)ethane (DPA2) and 1,3-P,P'-di(2-phospha-1,3,5,7-tetramethyl-6,9,10-trioxatricyclo[3.3.1.1{3.7}decyl)propane (DPA3) are used.

For the preparation of the catalyst system to be used in the process of the invention, the amount of bidentate ligand is suitably applied in some excess of the amount of platinum, expressed as moles of bidentate ligand per mole atom of platinum. The active species, however, is believed to be based on an equimolar amount of bidentate ligand per mole platinum. Thus, the molar amount of bidentate ligand per mole of platinum is suitably in the range of 1 to 3, preferably in the range of 1 to 2.

As regards the source of platinum, i.e., component (a) of the catalyst system, any platinum compound allowing complexing between the metal and the bidentate ligand may be used. Suitable compounds are for instance, metallic platinum, zerovalent platinum complexes, such as tetrakis(triphenylphosphine)platinum; and tetra- or divalent platinum salts. In particular platinum(II) salts are suitable, such as dipotassium tetracyanoplatinate, disodium tetracyanoplatinate, dipotassium tetrachloroplatinate, potassium trichloro (ethylene) platinate, sodium trichloro (ethylene) platinate, platinum-bis(cyanobenzene) disulphate and platinum-bis(triphenylphosphine) disulphate. Salts of platinum with carboxylic acids, in particular with carboxylic acids having from 2 to 12 carbon atoms, are also suitable, for example platinum diacetate, platinum dipropionate and platinum dihexanoate. Organic platinum(II) complexes are preferably applied as source of platinum, platinum(II) acetylacetonate being particularly suitable.

The molar amount of anion per mole of platinum is conveniently selected in the range of 1 to 12. Preferably the anion is applied in a molar amount per mole of platinum in the range of 1 to 8.

The process of the invention is carried out with catalytic amounts of the catalyst system, i.e. per mole of acetylenically unsaturated compound, 10⁻⁸ to 10⁻¹ mole of platinum is present, preferably from 10⁻⁷ to 10⁻², on the same basis.

The catalyst systems used in the process of the invention comprises further a source of anions as component (b). It is believed that the size of the anion and the distribution of electric charge in the anion significantly contribute to the stability of the catalyst system. Preferably, anions are used that are the conjugated base of acids having a pKa (measured at 18 °C in water) of less than 4. Suitable anions include anions derived from Bronsted acids, in particular from carboxylic acids, such as 2,6-dichlorobenzoic acid, and 2,6-bis(trifluoromethyl)benzoic acid or trifluoroacetic acid; and from sulphonic acids, such as methanesulphonic acid, and trifluoromethanesulphonic acid.

The acetylenically unsaturated compounds which may suitably be employed as starting material in the process of the invention, include compounds containing from 2 to 20 carbon atoms, optionally containing one or more inert substituents, such as halogen atoms or hydroxygroups. Preferably, the acetylenically unsaturated compounds has from 2 to 8 carbon atoms per molecule. The acetylenically unsaturated bond is usually the only carbon-carbon unsaturation in the molecule. In view of the envisaged preparation of mainly linear carbonylated products, it is preferably located at a terminal position. Examples of suitable acetylenically unsaturated compounds are acetylene(= ethyne), methylacetylene (= propyne), 1-butyne, 1-pentyne, 1-hexyne, 1-octyne, phenylacetylene and 3-hydroxybutyne. Acetylene is most preferred.

Suitable co-reactants in the process of the invention are hydrogen-containing compounds whereby a carbon monoxide molecule and at least one acetylenically unsaturated compound can be inserted into the bond between the hydrogen atom and the molecule of the co-reactant. Examples thereof include nucleophilic compounds containing at least one mobile hydrogen atom.

Preferred nucleophilic compounds include: water and alcohols, e.g., monohydric alcohols, such as methanol, ethanol, isopropanol and 1-butanol, and polyhydric alcohols, such as ethyleneglycol, 1,4-butanediol and glycerol; thiols; primary or secondary amines or amides; phenols and carboxylic acids, for example acetic acid, pivalic acid and propionic acid. Monohydric alcohols having from 1 to 6 carbon atoms are preferred, in particular methanol and butanol.

Another category of suitable co-reactants comprises hydride sources such as molecular hydrogen and compounds capable of generating molecular hydrogen. In particular for embodiments of the process whereby an acetylenically unsaturated compound is hydroformylated, molecular hydrogen is a preferred co-reactant.

Finally, also a combination of a nucleophilic compound and a hydride source as defined above may be used, to prepare a carbonylation-hydroformylation product.

The carbonylation process of the invention is generally carried out at a reaction temperature in the range of 40 to 200 °C, more suitably at a temperature in the range of 50 to 160 °C.

The total reaction pressure is usually in the range of 5 to 150 bar absolute (bara). Pressures between 10 and 80 bara and in particular between 30 and 60 bara are preferred. In carbonylation reactions of the hydroformylation type, the total pressure is usually the sum of the partial pressures of carbon monoxide and hydrogen. The molar ratio between these gases may vary, but is conveniently maintained in the range of 1:2 to 2:1. Preferably, substantially equimolar amounts of carbon monoxide and hydrogen are used. In other carbonylation reactions, involving no hydrogen, or only insignificant amounts thereof, the total pressure is roughly the same as the carbon monoxide pressure.

The process of the invention may be carried out in the absence of a separate diluent or solvent, if so desired. However, it is often convenient to have a liquid diluent or solvent present at the beginning of the reaction, e.g. if a volatile acetylenic starting material is used, or if the reaction requires a relatively long induction period and continued thorough mixing of the reactants is desirable. Suitable solvents are, in particular, aprotic compounds such as ethers or ketones, for example 2,5,8-trioxanonane, diethylether, acetone, diglyme and methyl ethylketone.

The invention is further illustrated by the following non-limiting examples.

### Example 1

(a) A 250 ml "Hastelloy C" (trademark) magnetically stirred autoclave was charged with acetylene (1.4 bara), 30 ml of 1-propanol, 0.1 mmol of Pt(acac)2, 0.12 mmol of DPA-3 ligand (see above), 0.25 mmol of methyl sulfonic-acid and 30 ml diglyme as solvent.

The autoclave was pressurised with 40 bara carbon monoxide and then sealed. The temperature of the mixture was raised to 115 °C. Complete conversion of acetylene was reached in shorter than 30 minutes at 115 °C and Glc analysis after cooling and depressurizing, showed a selectivity of 90 mol% to propyl acrylate. A propyl acrylate formation rate of 2000 t.o./hr/ mol Pt was calculated. Some byproducts are succinate and malonate diesters; these are due to consecutive carbonylation of acrylate at complete acetylene conversion. In a commercial operation the selectivity will be higher by keeping the acetylene conversion per pass below the 100%.

### Example 2

Example 1 was repeated except that the co-reactant was 30 ml methanol and that as acetylenically unsaturated compound 10 ml phenyl acetylene was used. The reaction time was 1 hour. A phenyl acrylate formation rate of 1000 turnovers per hour per mol Pt was calculated. The products consisted of 52% of linear 3-phenyl acrylate and 48% of 2-phenyl acrylate.

### Example 3

Example 2 was repeated except that as acetylenically unsaturated compound 10 ml 1-pentyne was used. The reaction time was 1 hour. A pentyne consumption rate of 300 turnovers per hour per mol Pt was calculated. The products consisted of 65% of linear methyl 2-hexenoate and 35% of 2-methyloxy carbonyl 1-pentene.

### Example 4

Example 1 was repeated except that as ligand DPA-2 (see above) was used and as co-reactant 30 ml methanol. A methyl acrylate formation rate of 290 t.o./hr/(mol Pt) was calculated. The reaction time was one hour and the selectivity towards methyl acrylate was 76 wt%.

## Claims

1. Process for the carbonylation of an acetylenically unsaturated compound by reaction with carbon monoxide and a co-reactant containing at least one mobile hydrogen atom in the presence of a catalyst system based on (a) a source of platinum; (b) a source of anions and (c) a diphosphine of the following formula
R¹>P-R²-PR³R⁴ (1)
wherein R² represents a covalent bridging group, R¹ represents a bivalent radical that together with the phosphorus atom to which it is attached is an optionally substituted 2-phospha-tricyclo[3.3.1.1{3,7}]decyl group or a derivative thereof in which one or more of the carbon atoms are replaced by heteroatoms, and wherein R³ and R⁴ independently represent univalent radicals of up to 20 atoms or jointly form a bivalent radical of up to 20 atoms.

2. Process according to claim 1, wherein R³ and R⁴ together with the phosphorus atom to which it is attached form an optionally substituted 2-phospha-tricyclo-[3.3.1.1{3,7}]decyl group or a derivative thereof in which one or more of the carbon atoms are replaced by hetero atoms, identical to the group formed by R¹ together with the phosphorus atom to which it is attached.

3. Process according to claim 2, wherein the optionally substituted 2-phospha-tricyclo[3.3.1.1{3,7}]decyl group or a derivative thereof in which one or more of the carbon atoms are replaced by hetero atoms is substituted on one or more of the 1, 3, 5 or 7 positions with a monovalent radical R⁵ of up to 20 atoms.

4. Process according to claim 3, wherein R⁵ is a radical selected from methyl, trifluoromethyl, ethoxy, phenyl, and 4-dodecylphenyl.

5. Process according to claim 4, wherein the optionally substituted 2-phospha-tricyclo[3.3.1.1{3,7}]decyl group or a derivative thereof in which one or more of the carbon atoms are replaced by hetero atoms is a 2-phospha-1,3,5,7-tetramethyl-6,9,10-trioxadamantyl group.

6. Process according to any one of claims 1-5, wherein R² has 2 to 4 atoms in the shortest chain of atoms directly connecting the two phosphorus atoms.

7. Process according to any one of claims 1-6, wherein the acetylenically compound has from 2 to 8 carbon atoms.

8. Process according to claim 7, wherein the acetylenically compound is acetylene.

9. Process according to any one of claims 1-8, wherein the co-reactant is a monohydric alcohols having from 1 to 6 carbon atoms.

10. Process according to any one of claims 1-9, wherein the source of anions is the conjugated base of acids having a pKa, as measured at 18 °C in water, of less than 4.

11. Process according to claim 10, wherein the acid is methanesulfonic acid.

## Patentansprüche

1. Verfahren zur Carbonylierung einer acetylenisch ungesättigten Verbindung durch Umsetzung mit Kohlenmonoxid und einer wenigstens ein mobiles Wasserstoffatom enthaltenden Co-Reaktante in Gegenwart eines Katalysatorsystems auf der Basis von (a) einer Platinquelle; (b) einer Anionenquelle und (c) eines Diphosphins der nachstehenden Formel
R¹>P-R²-PR³R⁴ (1),
worin R² eine kovalente Brückengruppe darstellt, R¹ für einen zweiwertigen Rest steht, der zusammen mit dem Phosphoratom, an das er gebunden ist, eine gegebenenfalls substituierte 2-Phospha-tricyclo[3.3.1.1{3,7}]decylgruppe oder ein Derivat hiervon darstellt, worin ein oder mehrere Kohlenstoffatome durch Heteroatome ersetzt sind, und worin R³ und R⁴ unabhängig voneinander einwertige Reste mit bis zu 20 Atomen darstellen oder gemeinsam einen zweiwertigen Rest mit bis zu 20 Atomen bilden.

2. Verfahren nach Anspruch 1, worin R³ und R⁴ zusammen mit dem Phosphoratom, an das sie gebunden sind, eine gegebenenfalls substituierte 2-Phosphatricyclo-[3.3.1.1{3,7}]decylgruppe oder ein Derivat hiervon ausbilden, worin ein oder mehrere Kohlenstoffatome durch Heteroatome substituiert sind, identisch zu der von R¹ zusammen mit dem Phosphoratom, an das es gebunden ist, gebildeten Gruppe.

3. Verfahren nach Anspruch 2, worin die gegebenenfalls substituierte 2-Phospha-tricyclo[3.3.1.1{3,7}]decylgruppe oder ein Derivat hiervon, worin ein oder mehrere Kohlenstoffatome durch Heteroatome ersetzt sind, in einer oder in mehreren der 1-, 3-, 5- oder 7-Positionen durch einen einwertigen Rest R⁵ mit bis zu 20 Atomen substituiert ist.

4. Verfahren nach Anspruch 3, worin R⁵ ein unter Methyl, Trifluormethyl, Ethoxy, Phenyl und 4-Dodecylphenyl ausgewählter Rest ist.

5. Verfahren nach Anspruch 4, worin die gegebenenfalls substituierte 2-Phospha-tricyclo[3.3.1.2{3,7}]decylgruppe oder ein Derivat hiervon, worin ein oder mehrere Kohlenstoffatome durch Heteroatome ersetzt sind, eine 2-Phospha-1,3,5,7-tetramethyl-6,9,10-trioxadamantylgruppe ist.

6. Verfahren nach einem der Ansprüche 1 bis 5, worin R² 2 bis 4 Atome in der kürzesten, die beiden Phosphoratome direkt verbindenden Kette von Atomen aufweist.

7. Verfahren nach einem der Ansprüche 1 bis 6, worin die acetylenische Verbindung 2 bis 8 Kohlenstoffatome aufweist

8. Verfahren nach Anspruch 7, worin die acetylenische Verbindung Acetylen ist.

9. Verfahren nach einem der Ansprüche 1 bis 8, worin die Co-Reaktante ein einwertiger Alkohol mit 1 bis 6 Kohlenstoffatomen ist.

10. Verfahren nach einem der Ansprüche 1 bis 9, worin die Anionenquelle die konjugierte Base von Säuren mit einem pKa-Wert, wie in Wasser bei 18°C bestimmt, von weniger als 4 ist.

11. Verfahren nach Anspruch 10, worin die Säure Methansulfonsäure ist.

## Revendications

1. Procédé de carbonylation d'un composé acétyléniquement insaturé par la réaction avec du monoxyde de carbone et un coréactif contenant au moins un atome d'hydrogène mobile en présence d'un système de catalyseur à base (a) d'une source de platine, (b) d'une source d'anions et (c) d'une diphosphine de la formule suivante :
R¹>P-R²-PR³R⁴ (1)
dans laquelle R² représente un groupe de pontage covalent, R¹ représente un radical bivalent qui, en même temps que l'atome de phosphore auquel il est fixé, représente un groupe 2-phospha-tricyclo[3.3.1.1{3,7}]décyle éventuellement substitué ou un dérivé de celui-ci dans lequel un ou plusieurs des atomes de carbone sont remplacés par des hétéroatomes, et dans lequel R³ et R⁴ représentent indépendamment des radicaux univalents comportant jusqu'à 20 atomes de carbone ou conjointement forment un radical bivalent comportant jusqu'à 20 atomes de carbone.

2. Procédé suivant la revendication 1, dans lequel R³ et R⁴ en même temps que l'atome de phosphore auquel ils sont fixés forment un groupe 2-phospha-tricyclo[3.3.1.1{3,7}]décyle éventuellement substitué ou un dérivé de celui-ci dans lequel un ou plusieurs des atomes de carbone sont remplacés par des hétéroatomes, identique au groupe formé par R¹ en même temps que l'atome de phosphore auquel il est fixé.

3. Procédé suivant la revendication 2, dans lequel le groupe 2-phospha-tricyclo[3.3.1.1{3,7}]décyle éventuellement substitué ou un dérivé de celui-ci dans lequel un ou plusieurs des atomes de carbone sont remplacés par des hétéroatomes, est substitué sur une ou plusieurs des positions 1, 3, 5 ou 7 par un radical monovalent R⁵ comportant jusqu'à 20 atomes de carbone.

4. Procédé suivant la revendication 3, dans lequel R⁵ est un radical choisi parmi le méthyle, le trifluorométhyle, l'éthoxy, lé phényle et le 4-dodécylphényle.

5. Procédé suivant la revendication 4, dans lequel le groupe 2-phospha-tricyclo[3.3.1.1{3,7}]décyle éventuellement substitué ou un dérivé de celui-ci dans lequel un ou plusieurs des atomes de carbone sont remplacés par des hétéroatomes, est un groupe 2-phospha-1,3,5,7-tétraméthyl-6,9,10-trioxadamantyle.

6. Procédé suivant l'une quelconque des revendications 1 à 5, dans lequel R² comporte de 2 à 4 atomes de carbone dans la chaîne d'atomes la plus courte reliant directement les deux atomes de phosphore.

7. Procédé suivant l'une quelconque des revendications 1 à 6, dans lequel le composé acétyléniquement insaturé comporte de 2 à 8 atomes de carbone.

8. Procédé suivant la revendication 7, dans lequel le composé acétyléniquement insaturé est de l'acétylène.

9. Procédé suivant l'une quelconque des revendications 1 à 8, dans lequel le coréactif est un alcool monoatomique comportant de 1 à 6 atomes de carbone.

10. Procédé suivant l'une quelconque des revendications 1 à 9, dans lequel la source d'anions est la base conjuguée d'acides ayant un pKa, tel que mesuré à 18°C dans l'eau, de moins de 4.

11. Procédé suivant la revendication 10, dans lequel l'acide est de l'acide méthanesulfonique.
